# EUROPÄISCHE PATENTANMELDUNG

(11) **EP 1 690 523 A1**
(43) Veröffentlichungstag der Anmeldung: **16.08.2006**
(21) Anmeldenummer: 06100212.7
(22) Anmeldetag: 10.01.2006
(51) Int. Cl.: A61K 8/27, A61K 8/29, A61K 8/04, A61Q 1/06, A61Q 1/08, A61Q 1/02, A61Q 1/12, A61Q 3/02

(54) **Kosmetische Zubereitungen zur dekorativen Anwendung enthaltend anorganische Gelbpigmente auf der Basis von Titan-, Zinn- und Zinkmischoxiden**

(30) Priorität: 11.02.2005 DE 102005007467
(71) Anmelder: Beiersdorf AG, 20253 Hamburg (DE)
(72) Erfinder: Lanzendörfer, Ghita, 90491, Nürnberg (DE); Riedel, Heidi, 22083, Hamburg (DE); Viehmeyer, Lynn, 20251, Hamburg (DE); Kallmayer, Volker, 22525, Hamburg (DE); Viala, Sophie, 20257, Hamburg (DE); Mocigemba, Nicole, 22529, Hamburg (DE)

(57) **Zusammenfassung**

Kosmetische Zubereitung zur dekorativen Anwendung auf Haut oder Nägeln, welche mindestens eine feste Phase enthält, die anorganische Pigmente aufweist, welche Titan-, Zinn- und Zinkmischoxide enthalten, wobei die molaren Mengenverhältnisse der Oxide der Summenformel (TiO₂)ₐSnO(ZnO)_{b}(SnO₂)_{c} entsprechen, worin a eine Zahl zwischen 0,3 und 6,2, b eine Zahl zwischen 0,04 und 6,2 und c eine Zahl zwischen 0 und 7 ist.

## Beschreibung

Die Erfindung betrifft kosmetische Zubereitungen zur dekorativen Anwendung auf Haut und Nägeln, die anorganische Gelbpigmente auf der Basis anorganischer Mischoxide aus Titandioxid, Zinn- und Zinkoxid enthalten.

Dekorative, kosmetische Zubereitungen sind farbige Zubereitungen, die das Gesicht, die Augenregion, die Lippen und die Nägel farblich verändern sollen. Unter dem Begriff Farbe wird im technisch-wissenschaftlichen Bereich ausschließlich der durch das menschliche Auge vermittelte Sinneseindruck verstanden. Die Strahlung zersetzt in den lichtempfindlichen Sinneszellen des Auges, den Zapfen, drei verschiedene Proteine mit spektral unterschiedlichen Absorptionsvermögen. Durch die Zersetzungsprodukte werden die Nervenverbindungen mit unterschiedlicher Stärke erregt. Für die Farbempfindung ist neben der spektralen Empfindlichkeit der Sehzapfen auch die neuronale Vernetzung in der Netzhaut des Auges verantwortlich.

Nur in seltenen Fällen wie z.B. bei der Flammenfärbung entsteht der Farbeindruck direkt aus der Emission. Demgegenüber erhalten gefärbte Körper ihre Farbe dadurch, dass sie einen Teil des auf sie fallenden weißen Lichts absorbieren, worauf der reflektierte Anteil des eingestrahlten Lichts dem Auge als Farbe des Körpers (Körperfarbe) erscheint.

Tabelle 1 führt die Wellenlängen der absorbierten Strahlung (in nm) zusammen mit der Farbe auf, die in Emission zu beobachten wäre und stellt diese der tatsächlichen beobachteten, reflektierten Körperfarbe gegenüber.

### Tabelle 1: Zusammenhang zwischen Wellenlänge der absorbierten Strahlung und

Farbeindruck

| absorbiertes Licht Wellenlänge in nm | Farbe bei Emission | Farbeindruck (beobachtete Farbe) |
|---|---|---|
| 730 | purpur (magenta) | grün |
| 640 | rot | blaugrün (cyan) |
| 590 | orange | blau |
| 550 | gelb | indigoblau |
| 530 | gelbgrün | violett |
| 510 | grün | purpur (magenta) |
| 490 | blaugrün (cyan) | rot |
| 450 | blau | orange |
| 425 | indigioblau | gelb |
| 400 | violett | grünlich-gelb |

### Quelle: Römpp Online, Version 2.5 (November 2004), Thieme Verlag, www.roempp.com

Als gelb wahrgenommene Körper, deren Farbton im Bereich zwischen orange über gelb bis grünlich-gelb liegen kann, absorbieren demnach Licht in einem Wellenlängenbereich von 400 bis 450 nm.

Um einen Farbeindruck zu erzeugen, enthalten dekorative Zubereitungen Farbmittel. Als Farbmittel finden Weiß- und Farbpigmente sowie Farbstoffe Verwendung. So werden vor allem Talkum, Zinkoxid, Kaolin, Titandioxid als Weißpigmente eingesetzt. Als Farbpigmente dienen anorganische Pigmente wie Eisenoxide (gelb bis rot, braun und schwarz), Chromoxide (grün), Ultramarin (blau, violett und rot) und Manganviolett (violett) sowie organische Pigmente. Auch werden Perlglanz-Pigmente wie Bismutoxidchlorid, Glimmer, mit Titandioxid beschichteter Glimmer und Fischsilber eingesetzt. In Lippenstiften werden zusätzlich geringe Mengen an Eosinfarben verwendet.

Im folgenden werden Pigmente, welche wie oben beschrieben eine Gelbfärbung bewirken, als Gelbpigmente bezeichnet. Die in kosmetischen Zubereitungen üblichen Gelbpigmente sind schwerlösliche Azoverbindungen und synthetische Eisenoxide. Hauptsächlich werden Eisenoxide verwendet. Sie sind toxikologisch unbedenklich, lichtecht und preiswert. Darüber hinaus zeigen sie ein sehr gutes Deckvermögen. Durch geeignete Mischungen lassen sich weitere Farbtöne einstellen. So ergeben Mischungen untereinander Brauntöne, Mischungen mit Titanoxid hingegen helle Farbtöne. Auf diese Weise lassen sich mit den üblichen Gelbpigmente ohne Probleme alle gewünschten Farbtöne einstellen.

Die Farbtiefe und Deckkraft einer pigmenthaltigen Formulierung werden unter anderem auch durch die Dispergierbarkeit des Pigments in der Formulierung bestimmt. Diese hängt von den physikalisch-chemischen Eigenschaften der Formulierung und vom Pigment selbst ab.

EP 0 113 229 beschreibt anorganische Komplexe aus Zinnoxid mit Titan- und Zinkoxiden, deren Herstellung und Verwendung. Diese Komplexe werden als Pigmente zum Anfärben von Gläsern und Keramiken eingesetzt. Sie sind auch zum Anfärben von organischen Polymeren, wie sie in Kunststoffen und Anstrichfarben eingesetzt werden, geeignet. In diesen Bereichen müssen Pigmente sehr temperaturstabil sein, d. h. sie dürfen bei den hohen Temperaturen, die zur Herstellung von Gläsern und Keramiken erforderlich sind, ihre Farbe nicht verändern oder verlieren. Bei Formverfahren für Kunststoffe, z.B. der Extrusion, können ebenfalls hohe Temperaturen auftreten. Auch müssen Pigmente in diesen Anwendungsbereichen - vor allem dann, wenn die Werkstoffe der Bewitterung ausgesetzt sind - ihre Farbe bei Sonneneinstrahlung und Feuchtigkeit über lange Zeit behalten. Pigmente, die diesen Anforderungen entsprechen und daher in diesen Bereichen üblicherweise eingesetzt werden, enthalten Schwermetalle wie Antimon, Blei oder Quecksilber.

Die anorganische Komplexe, die EP 0 113 229 beschreibt, basieren auf Komplexen aus Zinnoxid mit Titan- und Zinkoxiden. Sie sind frei von den oben erwähnten Schwermetallen. Die molaren Mengenverhältnisse der Oxide können durch die folgende Summenformel wiedergegeben werden:

(TiO₂)ₐSnO(ZnO)_{b}(SnO₂)_{c}

wobei
- a:: 0,3 bis 6,2
- b:: 0,04 bis 6,2
- c:: 0 bis 7

Die Komplexe können je nach Zusammensetzung unterschiedlich gefärbt sein und als gelbes, rotes, grünes oder blaues Pigment eingesetzt werden. Die Pigmente sind insbesondere bei warmen Farbtönen wie gelb und rot in ihrer Leuchtkraft und Farbe vergleichbar mit den bekannten schwermetallhaltigen, anorganischen Pigmenten.

Die Anforderungen an Pigmente für Kunststoffe, Farben, Gläser oder Keramik sind sehr verschieden von der Anforderungen an Pigmente für Kosmetika. So spielt die Temperatur- und Bewitterungsstabilität bei kosmetischen Zubereitungen keine Rolle. Die Pigmente müssen hier gut hautverträglich sein, mit Wasser und den üblichen Ölen, Fetten und Wachsen kompatibel sein und in Emulsionen dispergierbar sein. Die Pigmente dürfen sich in den meist flüssigen oder halbflüssigen Zubereitungen auch über längere Zeiträume nicht absetzen.

Auch zeigen die Inhaltsstoffe kosmetischer Zubereitungen ein völlig unterschiedliches physiko-chemischen Verhalten als die Inhaltsstoffe von Kunststoffen, Anstrichfarben, Gläsern und Keramiken.

Völlig unerwartet konnte gezeigt werden, dass Gelbpigmente auf der Basis von Titan-, Zinn- und Zinkmischoxiden, wie sie die Druckschrift EP 0 113 229 nennt, auch für kosmetische Formulierungen geeignet sind.

Die erfindungsgemäßen Zubereitungen ergeben überraschenderweise stabile, feinteilige Dispersionen, welche einen gleichmäßig deckenden Auftrag auf der Haut ermöglichen. Die Gelbpigmente auf Basis von Zinn-, Titan- und Zinkmischoxiden lassen sich leicht mit den üblichen Verfahren in die erfindungsgemäßen Zubereitungen einarbeiten. Die erhaltenen Zubereitungen zeigen darüber hinaus eine erheblich bessere Leuchtkraft und Farbintensität als Zubereitungen mit den üblichen Eisenoxid-Pigmenten.

Die Zubereitungen im Sinne der vorliegenden Erfindung können fest, flüssig oder halbfest sein. Sie enthalten mindestens eine Phase, in welcher die Feststoffe, Pigmente und gegebenenfalls Füllstoffe, dispergiert sind (Pigmentphase). Die Zubereitungen enthalten 0,01 bis 20 Gew.-%, bevorzugt 0,05 bis 15 Gew.-%, besonders bevorzugt 0,1 bis 12 Gew.-%, Gelbpigmente.

Für die erfindungsgemäßen Zubereitungen geeignete Gelbpigmente auf Basis von Titan-, Zinn- und Zinkmischoxiden sind z. B. SOLAPLEX-Pigment (Hersteller: Johnson Matthey Plc., London). Bevorzugt sind folgende SOLAPLEX-Pigmente: SOLAPLEX YELLOW, SOLAPLEX MID YELLOW und SOLAPLEX ORANGE.

Die erfindungsgemäßen Zubereitungen können zusätzlich weitere hydrophobe und hydrophile Phasen enthalten. Vorteilhafte, bei Raumtemperatur flüssige Phasen sind beispielsweise organische Lösungs- und Dispersionsmittel, Wasser oder kosmetisch verwendbare Öle.

Vorteilhafte organische Lösungs- und Dispersionsmittel sind beispielsweise Alkohole und Ester. Die Alkohole sind insbesondere einwertige Alkohole wie z.B. Ethanol oder Polyole wie z.B. Glycerin. Weitere vorteilhafte Polyole sind Zucker und ihre Derivate, Glykole, Polyethylenglykole, Methylpropandiol, Propylenglykol, Butylenglykol. Weiterhin kann es vorteilhaft sein Mischungen aus Alkoholen und Wasser einzusetzen. Besonders vorteilhafte Ester sind beispielsweise Ethyl- und Butylacetat.

Vorteilhafte Öle sind z.B. die folgende Substanzen und deren Mischungen:
- Mineralöle und andere Kohlenwasserstoffe wie z.B. Hexadecan, Isohexadecan, Dodecan, Isododekan, Eicosan oder Isoeicosan,
- Ester aus organischen Säuren mit 3 bis 30 Kohlenstoffatomen, die verzweigt oder unverzweigt, gesättigt oder ungesättigt sein können, gegebenenfalls mit zusätzlichen Hydroxygruppen, und Alkoholen mit 2 bis 30 Kohlenstoffatomen, die 1 bis 5 Hydroxygruppen enthalten können und die verzweigt oder unverzweigt, gesättigt oder ungesättigt sein können, wobei die Säuren ganz oder teilweise verestert sind wie z.B. Capryl-/Caprinsäuretriglyceride, Pentaerythrittri/tetraisostearat, Isopropylpalmitat, Sheabutter, Cetylactat, Methylpalmitat, Jojobaöl oder Sojaöl,
- Ether aus Alkoholen mit 2 bis 30 C-Atomen, die 1 bis 5 Hydroxygruppen enthalten können und die verzweigt oder unverzweigt, gesättigt oder ungesättigt sein können, wobei die Alkohole ganz oder teilweise verethert sein können, wie z.B. Dicaprylylether,
- Ester der Kohlensäure mit Alkoholen mit 2 bis 30 C-Atomen, die 1 bis 5 Hydroxygruppen enthalten können und die verzweigt oder unverzweigt, gesättigt oder ungesättigt sein können, wie z.B. Dicaprylylcarbonat,
- zyklische oder lineare, verzweigte oder unverzweigte Silikonöle sowie
- Ester aromatischer Carbonsäuren wie z.B. C₁₂₋₁₅-Alkylbenzoat.

Diese Substanzen können synthetisch oder natürlichen Ursprungs und gegebenenfalls raffiniert sein. Natürliche Öle, Fette und Wachse enthalten oft vorteilhafte Minderkomponenten wie Vitamine, Phytosterole und dergleichen. Besonders geeignete Substanzen können bei Raumtemperatur flüssig, halbfest oder fest sein und scharfe Schmelzpunkte oder breite Schmelzbereiche aufweisen. Die Ölphasen der erfindungsgemäßen Zubereitungen enthalten vorteilhafterweise die oben genannten Öle.

Weitere vorteilhafte erfindungsgemäße Zubereitungen enthalten neben der Pigmentphase eine oder mehrere hydrophobe Phasen (Ölphasen), welche Öle, Fette und Wachse enthalten können, und zusätzlich eine oder mehrere hydrophile Phasen (Wasserphasen), welche Wasser enthalten können . Sie können beispielsweise in Form von W/O-, O/W-, W/O/W- oder O/W/O-Emulsionen vorliegen. Darüber hinaus können solche Systeme zusätzlich in Öl- oder Wasserphasen dispergierte Feststoffe oder Gasblasen enthalten. Die erfindungsgemäßen Zubereitungen können in Form von Stiften, Schäumen (sog. Mousse), Feststoff-Emulsionen (d. h. Emulsionen, welche durch Feststoffe stabilisiert sind wie z. B. Pickering-Emulsionen), sprühbaren Emulsionen oder Hydrodispersionen vorliegen.

Als Feststoffe können beispielsweise Wachse oder kosmetisch verwendbare Puder- und Füllstoffe eingesetzt werden.

Vorteilhafte Wachse sind z.B. Bienenwachs, Teilfraktionen des Bienenwachs und Bienenwachsderivate, mikrokristalline Wachse, Ceresin, Ozokerite, Carnaubawachs, Candelillawachs, Reiswachs, Japanwachs, Schellackwachs und homopolymere Polyethylene.

Für die erfindungsgemäßen Zubereitungen geeignete Puder- und Füllstoffe sind z.B. Talkum, Glimmer (Mica), Nylon, pyrogene Kieselsäure, Kaolin, partikuläres Polyethylen sowie Stärke und Stärkederivate wie Aluminium- oder Natrium-Stärke-Octenylsuccinat und Distärkephosphat.

Vorteilhafte erfindungsgemäße Zubereitungen sind Nagellacke. Sie weisen neben Pigmenten üblicherweise Filmbildner, Glanz- und Haftungsverbesserer, Weichmacher, Lösungs- und Dispersionsmittel sowie weitere Hilfsstoffe auf.

Vorteilhafte organische Lösungs- und Dispersionsmittel sind beispielsweise Alkohole wie Isopropylalkohol oder n-Butylalkohol und Ester wie Ethylacetat und Butylacetat sowie Toluol. Ein geeigneter Filmbildner ist beispielsweise Nitrocellulose. Diese wird in der Regel mit Isopropanol versetzt (angefeuchtet), um die Explosionsgefahr zu verringern. Es ist vorteilhaft Dibutylphthalat, Campher oder Acetyltributyltricitrat z.B. als Weichmacher einzusetzen, um die Eigenschaften des Films zu verbessern. Die Zubereitung kann vorteilhafterweise Toluolsulfonamid-Formaldehyd-Harze, Polyacrylsäureester, Polymethacrylsäureester oder Mischungen daraus enthalten, um beispielsweise die Haftung des Films auf dem Nagel und den Glanzgrad des Films zu verbessern. Gegebenenfalls kann der erfindungsgemäße Nagellack weitere Hilfsstoffe wie Thixotropiermittel oder Verdicker (z.B. Stearalkoniumhektorit) und Lichtschutzmittel (z.B. Benzophenon-Derivate) enthalten. Der Nagellack kann darüber hinaus auch Wirkstoffe enthalten, die zur Nagelpflege geeignet sind.

Bei der Herstellung wird in der Regel das Lösungsmittel bzw. das Lösungsmittelgemisch vorgelegt und dann mit Nitrocellulose, den Kofilmbildnern, Harzen, Weichmachern und Verdickern eine Basis erstellt. Zu dieser werden dann die Pigmente, meist in Chipform, und die anderen Inhaltsstoffe gegeben.

Weitere erfindungsgemäße Zubereitungen sind Gele. Die Gelbpigmente auf Basis der Titan-, Zinn- und Zinkmischoxide können auch in einem eher polaren Medium wie Wasser dispergiert werden, wenn geringe Mengen oberflächenaktiver Substanzen wie z.B. PEG-40 Hydrogenated Castor Oil eingesetzt werden. Bei der Zugabe eines hydrophilen Verdickers werden Gele erhalten.

Bevorzugte erfindungsgemäße Zubereitungen, welche als zusätzliche feste Phase Wachse enthalten, sind z.B. Massen, die in Formen, insbesondere Stiftformen und Pfännchen, gegossen werden, und Pasten.

Technisch betrachtet sind die erfindungsgemäßen Massen wasserfreie Fettmischungen aus festen oder halbfesten Wachsen und flüssigen Ölen, wobei das Verhältnis von Wachs zu Öl die Härte der Masse und den Abrieb auf der Haut bestimmt. Über die Änderung dieses Verhältnisses kann die Härte und der Abrieb entsprechend eingestellt werden. So liegt z.B. für Lippenstifte das Verhältnis von Wachsen zu Ölen üblicherweise zwischen 10 Gew.-% zu 90 Gew.-% und 25 Gew.-% zu 75 Gew.-%.

Bevorzugte Grundstoffe für die erfindungsgemäßen Massen sind beispielsweise feste Bestandteile wie Bienenwachs, Ceresin oder mikrokristalline Wachse bzw. Ozokerit und hochschmelzende Wachse wie Carnaubawachs oder Candelillawachs, halbfeste Bestandteile wie Vaseline oder Lanolin und flüssige Öle wie Paraffinöle, Ricinusöl, Isopropylmyristat, C₁₂₋₁₅ Alkylbenzoat oder Diisostearylmaleat.

Zur Herstellung der erfindungsgemäßen Massen werden die Pigmente üblicherweise zunächst in einem Teil der Ölphase dispergiert. Anschließend werden die restlichen Rezepturbestandteile bevorzugt unter Erwärmung auf Temperaturen zwischen 70 und 100°C aufgeschmolzen und vermischt. Die Masse wird anschließend in entsprechende Formen gegossen. Aufgrund der relativ hohen Temperaturen ist die erfindungsgemäße Stiftmasse oxidationsgefährdet, daher werden üblicherweise Antioxidantien zugesetzt. Auch wird auf den Einsatz oxidationsempfindlicher Inhaltsstoffe verzichtet.

Bei der Herstellung von Lippenstiften werden die Stifte nach dem Erkalten in Hülsen verbracht. Andere Zubereitungen wie z.B. gegossene Blushes verbleiben in der Form.

Zu den Pasten im Sinne der Erfindung gehören z.B. wasserfeste Maskaras. Die erfindungsgemäßen Maskaras sind bevorzugt Dispersionen von Pigmenten in einem Wachs/Öl-Gemisch, bei denen das Öl ein bei Raumtemperatur flüchtiges Öl ist, wie z.B. Isododecan oder Cyclomethicone. Die Aufgabe der Wachse ist es, der erfindungsgemäßen Zubereitung eine Konsistenz zu verleihen, die eine optimale Entnahmemenge bezogen auf die Art der Verpackung (Schraubflasche mit Bürste) gewährleistet. Darüber hinaus verhindern die Wachse das Ausölen der flüchtigen Komponenten und bilden einen Film auf den Wimpern. Es werden bevorzugt Mischungen von Wachsen verwendet. Diese werden aus der Gruppe bestehend aus Bienenwachsen, Teilfraktionen des Bienenwachs oder Bienenwachsderivaten, Reiswachs, Candellilawachs, Carnaubawachs, Japanwachs, Schellackwachs, Kohlenwasserstoffwachsen aus der Gruppe der Paraffine, der mikrokristallinen Wachse und der Ozokerite sowie homopolymeren Polyethylenen gewählt.

Erfindungsgemäße Zubereitungen, die neben der Pigmentphase Öl- und Wasserphasen enthalten (Öl/Wasser-Systeme) sind beispielsweise Emulsionen oder Hydrodispersionen. Emulsionen im Sinne der Erfindung können O/W-, W/O-, W/Si- (Wasser-in-Silikonöl), O/W/O-, W/O/W- oder Feststoff-Emulsionen sein. Diese Emulsionen können auch fest, sprühbar oder fließfähig sein. Erfindungsgemäße Emulsionen enthalten neben der Ölphase, die aus Ölen, Fetten, Wachsen oder deren Mischungen bestehen kann, Wasser und vor allem Emulgatoren sowie gegebenenfalls weitere Hilfsstoffe wie Coemulgatoren, Konsistenzgeber, Verdicker, Stabilisatoren, Polyole, Hydrotrope, Konservierungsmittel, Wirkstoffe, UV-Filter sowie Puder- und Füllstoffe.

So enthalten erfindungsgemäße Emulsionen O/W-Emulsionen mindestens einen Emulgator mit einem HLB Wert > 7 und gegebenenfalls einen Coemulgator. Diese Emulgatoren können vorteilhaft gewählt werden aus der Gruppe der nichtionischen oder anionischen Emulgatoren.

Unter den nichtionischen Emulgatoren befinden sich vorzugsweise
a) Partialfettsäureester und Fettsäureester mehrwertiger Alkohole und deren ethoxilierte Derivate (z. B. Glycerylmonostearate, Sorbitanstearate, Glyceryl Stearyl Citrate, Sucrose Stearate) und
b) ethoxilierte Fettalkohole und Fettsäuren.

O/W-Emulgatoren, mit denen sich erfindungsgemäße O/W-Emulsionen herstellen lassen, sind vor allem nichtionische oder anionische Emulgatoren. Vorteilhafte nichtionische O/W-Emulgatoren sind ethoxylierte Fettalkohole oder Fettsäuren, bevorzugt PEG-100-Stearat, PEG-40-Stearat, Ceteareth-20, Ceteth-20, Steareth-20, Ceteareth-12, Ceteth-12, Steareth-12 sowie Ester aus Mono-, Oligo- oder Polysacchariden mit Fettsäuren, bevorzugt Cetearylglucosid und Methylglucosedistearat.

Vorteilhafte anionische Emulgatoren sind Seifen (z. B. Natrium- oder TriethanolaminSalze der Stearin- oder Palmitinsäure) sowie Ester der Zitronensäure wie Glycerylstearatcitrat.

Als geeignete Coemulgatoren für die erfindungsgemäßen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglykolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.

Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol, Polyethylenglykol(2)stearylether (Steareth-2).

Es kann im Sinn der vorliegenden Erfindung vorteilhaft sein, weitere Emulgatoren zu verwenden. So kann beispielsweise die Wasserfestigkeit der erfindungsgemäßen Zubereitungen erhöht werden. Geeignete Emulgatoren sind z.B. Alkylmethiconcopolyole und Alkyl-Dimethiconcopolyole, insbesondere Cetyldimethiconcopolyol, Laurylmethiconcopolyol, W/O-Emulgatoren wie Sorbitanstearat, Glycerylstearat, Glycerolstearat, Sorbitanoleat, Lecithin, Glycerylisostearat, Polyglyceryl-3-oleat, Polyglyceryl-3-diisostearat, PEG-7-hydriertes Rizinusöl, Polyglyceryl-4-isostearat, Acrylat/ C₁₀₋₃₀-Alkylacrylat-Crosspolymer, Sorbitanisostearat, Poloxamer 101, Polyglyceryl-2-dipolyhydroxystearat, Polyglyceryl-3-diisostearat, Polyglyceryl-4-dipolyhydroxystearat, PEG-30-dipolyhydroxystearat, Düsostearoylpolyglyceryl-3-diisostearat, Glykoldistearat, Polyglyceryl-3-dipolyhydroxystearat.

Zur Stabilisierung der erfindungsgemäßen O/W-Emulsionen können hydrophile Gelbildner eingesetzt werden. Vorteilhafte Gelbildner für derartige Zubereitungen sind beispielsweise Copolymere aus C₁₀₋₃₀-Alkylacrylaten und einem oder mehreren Monomeren der Acrylsäure, der Methacrylsäure oder deren Ester. Die INCI-Bezeichnung für solche Verbindungen ist "Acrylates/C 10-30 Alkyl Acrylate Crosspolymer". Insbesondere vorteilhaft sind Pemulen TR 1, Pemulen TR 2 und Pemulen TRZ von der Fa. Noveon.

Auch Carbomere sind vorteilhafte Gelbildner für derartige Zubereitungen. Carbomere sind Polymere der Acrylsäure, insbesondere auch Acrylat-Alkylacrylat-Copolymere. Vorteilhafte Carbomere sind beispielsweise Carbopol 980, Carbopol 981, Carbopol 1342, Carbopol 1382, Carbopol 2984 und Carbopol 5984 sowie Carbopol ETD 2020, Carbopol ETD 2050 und Carbopol Ultrez 10 der Fa. Noveon. Weitere vorteilhafte Gelbildner für derartige Zubereitungen sind Xanthangummi, Cellulosederivate oder Johannisbrotkernmehl sowie Acrylamidmethylpropylsulfonsäurepolymere (so genannte AMPS-Polymere) insbesondere hydrophobisierte Acrylamidomethylpropylsulfonsäure-Polymere, die am AMPS-Grundgerüst über PEG-Ketten (mit 2 bis 200, bevorzugt 5 bis 25 Ethoxyeinheiten) gebundene Alkylketten tragen, wobei die Kettenlänge im Bereich zwischen C₆ und C₂₂ liegt.

Zur Verbesserung des Hautgefühls und der Stabilität sowie zur Einstellung der Viskosität können Verdicker und Konsistenzgeber zugesetzt werden. Besonders vorteilhafte Konsistenzgeber sind hierbei Glycerylstearat, -palmitat oder -oleat sowie Fettalkohole, vor allem unverzweigte gesättigte Fettalkohole mit 10 bis 24 C-Atomen.

Weiterhin vorteilhaft können Filmbildner enthalten sein, beispielsweise um eine gleichmäßigere Verteilung der Pigmente auf der Haut zu erhalten und um die Transferresistenz der auf der Haut aufgetragenen Zubereitung zu verbessern. Als hydrophile Filmbildner kommen z.B. Polyurethane-1 (Luviset PUR, BASF), Polyvinylcaprolactam (Luviskol Plus, BASF), Acrylates Copolymer (Luviflex Soft, BASF), PVP/PA Copolymer (Luviskol VA 64W, BASF oder PVP/VA W-635/735, ISP), PVP (z.B. Luviskol K 30, BASF) Diglycol/CHDM/Isophthalates/SIP Copolymer (AQ 48 Ultra, Eastman) und Chitosan in Frage.

Eine vorteilhafte erfindungsgemäße O/W-Emulsion ist beispielsweise eine O/W-Maskara. Es handelt sich hierbei bevorzugt um ein mit Triethanolamin verseiftes Stearatsystem, zu dem noch weitere Emulgatoren gegeben werden können, mit hohen Mengen an Wachsen, Pigmenten und Filmbildnern. Außer Triethanolamin kann auch Aminomethylpropanol als Verseifungsmittel für die Stearinsäure verwendet werden. Vorzugsweise liegt die Einsatzkonzentration von Triethanolamin-Stearat bei 5 bis 8 Gew.-%, die der Pigmente bei 8 bis 12 Gew.-% und die der Wachse bei 8 bis 15 Gew.-%. Als Filmbildner können sowohl hydrophile als auch lipophile Filmbildner eingesetzt werden. Weiterhin ist der Einsatz von Verdickern wie z.B. Hydroxyethylcellulose sinnvoll.

Wachse können aus der Gruppe der Bienenwachse und ihrer Teilfraktionen sowie der Bienenwachsderivate, der Gruppe der paraffinischen Kohlenwasserstoffwachse wie mikrokristalline Wachse und Ozokerite sowie aus der Gruppe der homopolymeren Polyethylene gewählt werden. Ebenfalls vorteilhaft können natürliche Wachse wie Reiswachs, Candellilawachs, Carnaubawachs, Japanwachs oder Schellackwachs eingesetzt werden.

Erfindungsgemäße Zubereitungen können W/O- bzw. Wasser-in-Silikonöl- (W/S-) Emulsionen sein. Vorteilhaft im Sinn der Erfindung sind dabei W/O- bzw. W/S-Emulsionen , die
- ein oder mehrere Silikonemulgatoren (W/S) mit einem HLB-Wert ≤ 8 oder
- ein oder mehrere W/O-Emulgatoren mit einem HLB-Wert < 7 oder eine Mischung dieser Emulgatoren und gegebenenfalls
- ein oder mehrere O/W-Emulgatoren mit einem HLB-Wert > 10 enthalten.

Die erfindungsgemäßen W/O-Emulsionen enthalten vorteilhaft mindestens 20 Gew.-% an Ölphase, wobei die Ölphase Silikonöle enthalten oder ganz aus Silikonölen (W/Si-Emulsionen) bestehen kann. Weiterhin können die erfindungsgemäßen W/O-Emulsionen Feststoffe in einem Bereich von 5 bis 25 Gew.-% enthalten, wobei sich die Feststoffphase bevorzugt aus Pigmenten und Füllstoffen zusammensetzt.

Die Silikonemulgatoren können vorteilhaft aus der Gruppe der Alkyl-Dimethiconcopolyole z.B. Cetyl PEG/PPG 10/1 Dimethiconcopolyol (ABIL® EM 90 der Fa. Goldschmidt AG), Lauryl PEG/PPG- 18/18 Dimethicone (Dow Corning® 5200 Formulation Aid der Fa. Dow Corning Ltd.) oder Dimethiconecopolyole z.B. PEG-10 Dimethicone (KF-6017 der Fa. Shin Etsu), PEG/PPG- 18/18 Dimethicone (Dow Corning Formulation aid 5225C der Fa. Dow Corning Ltd.), PEG/PPG-19/19 Dimethicone (Dow Corning BY-11 030 der Fa. Dow Corning Ltd.) gewählt werden.

Die W/O-Emulgatoren mit einem HLB-Wert < 7 können vorteilhaft aus der Gruppe bestehend aus Sorbitanstearat, Sorbitanoleat, Glycerylisostearat, Polyglyceryl-3-oleat, Pentaerythrithylisostearat, Methylglucosedioleat, PEG-7-hydriertes Ricinusöl, Polyglyceryl-4-isostearat, Hexyllaurat, Sorbitanisostearat, Polyglyceryl-2-dipolyhydroxystearat, Polyglyceryl-3-diisostearat, PEG-30-dipolyhydroxystearat, Diisostearoylpolyglyceryl-3-diisostearat, Polyglyceryl-3-dipolyhydroxystearat, Polyglyceryl-4-dipolyhydroxystearat, Polyglyceryl-3-dioleat und Wollwachsalkohol (Eucerit) gewählt werden.

Die O/W-Emulgatoren mit einem HLB-Wert > 10 können vorteilhaft aus der Gruppe bestehend aus Lecithin, Trilaureth-4-phosphat, Polysorbate-20, Polysorbate-60, PEG-22-Dodecylglykol Copolymer, Sucrosestearat, Sucroselaurat gewählt werden.

Zur Stabilisierung der erfindungsgemäßen Emulsionen gegen Sedimentierung oder Flockung der Wassertröpfchen kann es von Vorteil sein, darüber hinaus noch Ölverdicker hinzuzusetzen. Besonders geeignet sind hierfür Aerosil (ggf. modifizierte pyrogene Kieselsäure), Silikate wie Bentonit, Hectorit oder Montmorillonit (zur Optimierung der Eigenschaften ggf. oberflächenbehandelt) sowie Magnesium- oder Aluminiumstearat.

Weiterhin vorteilhaft kann der Einsatz von Wachsen sein, insbesondere von silikonmodifizierten Wachsen wie z.B. Cetearyl Methicone, Siliconyl Beeswax oder C30-45 Alkyl Dimethicone.

Ebenso können der Ölphase der erfindungsgemäßen Emulsionen weitere Silikonöle, silikonmodifizierte Öle zugesetzt werden. Als vorteilhafte Silikonöle können Dimethicone, Phenyl Trimethicone oder Bis-Phenylpropyl Dimethicone eingesetzt werden. Vorteilhafte silikonmodifizierte Öle sind z.B. Methyl Ester Siloxane (SF 1318 der Fa. GE Bayer) oder Phenylpropyl Dimethylsiloxysilicat (Silshine 151 der Fa. GE Bayer).

Die erfindungsgemäße Emulsion kann zusätzlich Filmbildner enthalten, um eine gleichmäßigere Verteilung der Pigmente auf der Haut zu erhalten und um die Transferresistenz der auf der Haut aufgetragenen Zubereitung zu verbessern. Vorteilhafte lipophile Filmbildner sind z.B. Trimethylsiloxysilicate (z.B. SR 1000 der Fa. GE Bayer), Silikon Acrylate Copolymere (z.B. TIB4-200 der Fa. Dow Corning, oder KP-561 der Fa. Shin Etsu) oder Trimethyl Pentaphenyl Trisiloxane (555 Cosmetic Fluid der Fa. Dow Corning).

Darüber hinaus kann es von Vorteil sein, der Rezeptur nicht-partikuläre sensorische Additive zuzusetzen, welche gewählt werden können aus der Gruppe der Dimethiconole (z.B. Dow Corning 1503 Fluid), der Siliconcopolymere (z.B. Divinyldimethicone/Dimethicone Copolymer, Dow Corning HMW 2220) oder der Siliconelastomere (z.B. Dimethicone Crosspolymer, Dow Corning 9040 Silicone Elastomer Blend).

Weitere Hilfsstoffe, die sowohl in W/O- als auch in O/W-Emulsionen eingesetzt werden können, sind beispielsweise Konservierungsmittel, Puder- und Füllstoffe, Hydrotrope und Wirkstoffe.

Geeignete Konservierungsmittel für Emulsionen sind Parabene, Phenoxyethanol, lodopropinylbutylcarbamat, Sorbinsäure, DMDM Hydantoin, Diazolidinylharnstoff, Sorbinsäure (vor allem bei pH-Werten kleiner 5) und Benzylalkohol. Es ist bekannt, dass Mischungen von Konservierungsmitteln synergistische Effekte zeigen, weshalb in der Regel verschiedene Kombinationen mikrobiologischen Belastungstests unterzogen werden. Es kann darüber hinaus vorteilhaft sein, die Konservierungsmittel in anderen Stoffen gelöst einzusetzen, vor allem Polyole finden hier vorteilhaft Verwendung.

Geeignete Puder- und Füllstoffe sind insbesondere Stärke und Stärkederivate (z.B. Aluminum- oder Natrium-Stärke-Octenylsuccinat, Distärkephosphat), Talkum, Glimmer (Mica), Nylon (z.B. Nylon-12 oder Nylon-6/12), pyrogene Kieselsäuren, Kaolin oder partikuläres Polyethylen. Die Teilchengröße der Rohstoffe liegt bevorzugt zwischen 1 und 30 µm und ihr Brechungsindex ist vorteilhaft zwischen 1 und 5.

Geeignete Hydrotrope sind aufgrund ihrer guten Hautpflegeeigenschaften insbesondere wasserlösliche organische Verbindungen wie z.B. Ethanol, Isopropanol, Harnstoff, Ethylhexyloxyglycerin, Methylpropandiol, Glycerin oder Alkandiole mit 2 bis 10 Kohlenstoffatomen wie Propylenglykol, Butylenglykol usw.

Es sind zahlreiche Wirkstoffe bekannt, die auch in der vorliegenden Erfindung vorteilhaft zum Einsatz kommen können. Besonders geeignet sind Vitamine wie z.B. Vitamin A, B, C, E und deren Derivate und Provitamine sowie Antioxidantien wie z.B. Flavonoide, Isoflavonoide und deren Derivate.

Weitere vorteilhafte erfindungsgemäße Zubereitungen sind Puder. Puderformulierungen sind pulverförmige Zubereitungen, die in loser oder gepresster Form (Kompaktpuder oder Pudersteine) vorliegen können. Erfindungsgemäße Puderformulierungen finden als Gesichtspuder, Lidschatten oder Rouge Anwendung. Wesentliche Bestandteile sind pulverförmige Mineralien, besonders bevorzugt Talkum. Gegebenenfalls können weitere Stoffe zugesetzt werden, um einzelne Eigenschaften zu verbessern. So können beispielsweise Siliziumdioxid, Magnesiumcarbonat, Kaolin, Kreide oder Stärke eingesetzt werden, um das Aufsaugvermögen des Puders von Wasser und hydrophilen Stoffe oder von Ölen, Fetten oder lipophilen Stoffen zu verbessern. Titandioxid, Zinkoxid und Kreide können verwendet werden, um die Deckkraft und das Haftvermögen des Puders zu erhöhen. Das Haftvermögen lässt sich ferner durch Stärke, Aluminiumoxid, Zinkundecanat oder auch Metallseifen verbessern. Des weiteren kann durch den Einsatz von Zink-, Magnesium- und/oder Aluminiumsalzen der Laurin-, Myristin- und/oder Stearinsäure sowie durch Zink- und/oder Magnesiumdecanat oder Talk die Gleitfähigkeit des Puders gesteigert werden.

Besonders vorteilhafte erfindungsgemäße Puderzubereitungen sind Pudersteine oder Kompaktpuder. Sie enthalten die für lose Pudern üblichen Grundstoffe und zusätzlich einen Binder wie z.B. Magnesiumstearat. Die Pudermischung wird unter Druck (etwa 40 bar) in flache Formen oder Pfännchen (Godets) gepresst. Erfindungsgemäße Kompaktpuder können auch hergestellt werden, indem man die Grundstoffe mit einer kleinen Menge Wasser und Binder zu einem Teig verarbeitet, der in kleine Formen gegossen wird, die man dann trocknen lässt, so dass der Inhalt fest wird.

Die folgenden Beispiele sollen die Erfindung näher erläutern, ohne sie darauf zu beschränken.

### Beispiele

### 1. Gegossene Zubereitungen

### Beispiel 1a: Pflegende Lippenstifte

| **Inhaltsstoff (INCI-Bezeichnung)** | **Anteil in Gew.-%** | | | |
|---|---|---|---|---|
| | **1** | **2** | **3** | **4** |
| Rizinusöl | ad 100 | | 6,0 | 3,0 |
| Caprylic-/Capric Triglycerides | 20,0 | 20,0 | ad 100 | 3,0 |
| Pentaerythrityl Tetraisistearate | | 5,0 | | |
| Macadamiaöl | | | 2,0 | |
| Octyldodecanol | 20,0 | 5,0 | 6,0 | 6,0 |
| Hydrogenated Polyisobuten (z.B. Parleam Type 4, Rossow Cosmétiques) | | | 10,0 | 1,0 |
| Polyisobutene | | 2,0 | 3,0 | |
| Isopropylpalmitat | | 3,5 | 6,0 | 2,0 |
| Jojobaöl | 6,0 | | | 1,0 |
| Lanolin Öl | 5,0 | | 9,0 | 1,0 |
| PEG-45/ Dodecyl Glykol Copolymer | | | | 2,0 |
| Polyglyceryl-3 Diisostearat | 3,7 | 3,5 | | 2,4 |
| Bis-Diglyceryl Polyacyladipat-2 | | 6,0 | | |
| Cetearyl Alkohol | 6,0 | 0,5 | | |
| Cetylpalmitat | 7,5 | | | 1,0 |
| C20-40 Alkyl Stearat | 16,0 | | 3,0 | 8,0 |
| Carnaubawachs | | 5,0 | 4,0 | 2,0 |
| Candelillawachs | | 3,0 | 4,0 | |
| Bienenwachs | 6,0 | 2,0 | | |
| Mikrokristallines Wachs | | 8,0 | 4,0 | 8,0 |
| PVP / Eicosen Copolymer | | | 0,5 | 0,2 |
| 4-Methylbenzyliden Campher | | | | 5,0 |
| Octyl Methoxycinnamat | 3,0 | 3,0 | 3,0 | 2,5 |
| Ethylhexyl Triazon | | | 2,0 | 6,0 |
| Octocrylen | | 3,0 | | |
| Butyl Methoxydibenzoylmethan | 2,0 | | | 0,8 |
| Lauroyl Lysin | 0,5 | | 0,6 | |
| Pigment Yellow 216-Dispersion 40%ig (Solaplex, Johnson Matthey) | 0,5 | 2,6 | 4,2 | 6,0 |
| Titandioxid | | 0,6 | 1,0 | 2,0 |
| Eisenoxide | | 2,4 | 2,0 | 1,0 |
| Effektpigmente | | 6,0 | 3,5 | |
| Glimmer | | | 4,0 | |
| Diammonium Citrat | 0,08 | | | |
| Zitronensäure | 0,05 | | | |
| Glycerin | | | | 10 |
| Tocopheryl Acetate | 0,5 | | 0,5 | 1,0 |
| Ascorbyl Palmitate | | 0,2 | 0,3 | |
| Ubichinon | | 0,5 | | |
| Parfum, Konservierungsmittel, BHT, Neutralisationsmittel, Sequestriermittel | q. s. | q. s. | q. s. | q. s. |
| Wasser | 2,5 | | | ad 100 |

### Beispiel 1b: Langhaltende Lippenstifte

| **Inhaltsstoffe (INCI-Bezeichnung)** | **Anteil in Gew.-%** | | | | |
|---|---|---|---|---|---|
| | **1** | **2** | **3** | **4** | **5** |
| Mikrokristallines Wachs | 3,0 | 4,0 | | 2,5 | 8,0 |
| Polyethylenwachs | | 6,0 | 10,0 | 12,0 | |
| C30-C50 Fettalkohole | | | 10,0 | 6,0 | |
| Carnaubawachs | | | | | 2,0 |
| Candelillawachs | 12,0 | 2,0 | | 6,0 | 8,0 |
| Bienenwachs | 2,5 | | | | 1,0 |
| C20-40 Alkyl Stearat | | | | 1,5 | |
| Lanolinöl | | 3,0 | | 2,0 | |
| Bis-Diglyceryl Polyacyladipat-2 | | 5,0 | | | 6,0 |
| Polyglyceryl-3 Diisostearate | 10,0 | | | | |
| Cyclomethicone | 25,0 | | | 25,0 | 30,0 |
| Isododecan | | 50,0 | 45,0 | 10,0 | 5,0 |
| Jojobaöl | | | | | 3,0 |
| Octyldodecanol | ad 100 | | ad 100 | ad 100 | |
| Polybutene | 5,0 | | 3,0 | | 2,0 |
| Caprylic/Capric Triglycerides | 12,0 | ad 100 | | | ad 100 |
| Pentaerythrityl Tetraisostearate | 10,0 | | | | 3,0 |
| Acrylates Copolymere | | | 8,6 | 6,0 | |
| Styrene Copolymere | | | 2,0 | | 2,0 |
| Trimethylsiloxysilicate | | 0,5 | | 2,6 | 2,0 |
| PVP/Hexadecen Copolymer | 0,5 | | | 0,3 | |
| Silica Dimethyl Silylat (Aerosil R972) | | 0,5 | | 0,5 | 0,2 |
| Polymethylsilsesquioxan (Tospearl 2000B) | | | 3,0 | 3,0 | 3,0 |
| Disteardimonium Hectorite | | | 0,5 | | 0,5 |
| Propylen Carbonat | | | 0,12 | | 0,12 |
| Ethylhexyl Methoxycinnamat | 0,5 | 0,5 | | 2,0 | 0,5 |
| Butyl Methoxydibenzoylmethan | 0,2 | 0,2 | | | 0,2 |
| Bis-Ethylhexyloxyphenol Methoxyphenyl Triazin | | 2,0 | | 0,25 | |
| Mikronisiertes Titandioxid (Eusolex T 2000) | | 2,0 | | | 2,0 |
| Goniochromatische Pigmente | 5,0 | | | 6,0 | 8,0 |
| Metallpigmente | | | 2,0 | | 2,0 |
| Interferenz Pigmente | | 4,0 | 3,0 | 3,0 | |
| Titandioxid Cl 77891 | 1,0 | 3,8 | 2,0 | | |
| Eisenoxide CI 77491, 77492, 77499 | 0,6 | 2,2 | | | |
| CI 15850 | | | 1,9 | | |
| CI 42090 | 0,3 | | | 0,6 | |
| CI 19140 | | 0,6 | | | |
| CI 15985 | | | 1,0 | | |
| CI 45380 | | | | 0,6 | |
| CI 73360 | | 1,5 | | | |
| Pigment Yellow 216 (Solaplex, Johnson Matthey) | 6,3 | 0,6 | 2,5 | 2,5 | 5,0 |
| Tocopheryl Acetat | 1,0 | 2,0 | 0,5 | 1,5 | 1,0 |
| Konservierungsmittel, BHT, Parfum, Aroma | q. s. | q. s. | q. s. | q. s. | q. s. |

### Beispiel 1c: Hochglänzende Lippenstifte

| **Inhaltsstoff (INCI-Bezeichnung)** | **Anteil in Gew.-%** | | | | |
|---|---|---|---|---|---|
| | **1** | **2** | **3** | **4** | **5** |
| Mikrokristallines Wachs | 5,0 | 8,0 | 3,5 | 2,0 | |
| Polyethylenwachs | 5,0 | 6,0 | 8,0 | 8,0 | 8,0 |
| C30-C50 Fettalkohole | | | 6,0 | 2,0 | 6,0 |
| Candelillawachs | 5,0 | 2,0 | | 2,0 | 2,0 |
| C20-40 Alkyl Stearat | | | | 0,5 | |
| Phenyltrimethicone | | 10,0 | | | 5,0 |
| Phenylpropyldimethylsiloxysilicate | 5,0 | | 10,0 | 15,0 | |
| Trimethyl Pentaphenyl Trisiloxane (DC Cosmetic Fluid 555) | 5,0 | | 10,0 | | 5,0 |
| Methyl Ether Siloxane (GE SF 1318) | 5,0 | | | 2,0 | 10,0 |
| Triisocetylcitrate | 10,0 | | | | 5,0 |
| Diisosteraryl Malate | 5,0 | | 10,0 | 8,0 | 5,0 |
| Lanolinöl | | 9,0 | | | 3,0 |
| Cocoglycerides | ad 100 | | ad 100 | 5,0 | ad 100 |
| Rizinusöl | | ad 100 | | ad 100 | 2,0 |
| Cetyl Ricinoleate | | 6,0 | | | 0,8 |
| Avocadoöl | 10,0 | 5,0 | | | |
| Isopropyl Palmitate | | | 6,0 | | 5,0 |
| Pentaerythrityl Tetraisostearat | | 2,0 | 10,0 | 5,0 | |
| Polyisobuten | | 5,0 | | 5,0 | |
| hydriertes Polydecen | | 13,0 | | 3,0 | |
| Polyglyceryl-3 Diisostearate | | | | 0,5 | |
| Ethylhexyl Methoxycinnamat | 3,00 | | 4,50 | | 8,50 |
| Bis-Ethylhexyloxyphenomethoxyphenyl Triazin | 0,50 | 3,00 | 2,50 | 1,00 | |
| Butyl Methoxydibenzoylmethan | | | | | 2,00 |
| Ethylhexyl Triazon | | 2,00 | | | |
| Octocrylen | 6,00 | | 3,00 | 2,00 | |
| Diethylhexyl Butamido Triazon | | | 1,50 | | |
| Methylen Bis-Benzotriazolyl Tetramethylbutylphenol | 3,50 | 0,75 | | | 2,00 |
| Diethylhexyl-2,6-naphthalat | | | | | 5,50 |
| Disteardimonium Hectorite | | 0,5 | | | |
| Propylen Carbonat | | 0,12 | | | |
| Silica Dimethyl Silylate (Aerosil R972) | | | | | 0,3 |
| Glimmer | | | | 5,0 | |
| Calcium Aluminium Borosilikat (Rona Star Nobel Sparks, Merck) | | | 10,0 | 6,0 | 3,0 |
| Interferenz Pigmente | 10,0 | 6,8 | 0,6 | | |
| Pigment Yellow 216 (Solaplex, Johnson Matthey) | 2,8 | 0,6 | 6,0 | 3,6 | 1,2 |
| Titandioxid CI 77891 | | 2,0 | | 2,0 | 2,0 |
| Eisenoxide CI 77491, 77492, 77499 | | | 0,6 | 1,5 | 2,0 |
| CI 15850 | 1,3 | 3,2 | | 1,5 | 0,5 |
| CI 42090 | | | 0,2 | | |
| CI 17200 | | 0,3 | | 0,75 | |
| CI 45380 | 0,1 | | | | 0,05 |
| CI 15985 | | | | | |
| CI 73360 | | 1,0 | | | |
| CI 45410 | | | | 0,5 | |
| Tocopheryl Acetate | 0,5 | | 1,0 | 1,5 | |
| Parfüm, Konservierungsmittel, Filmbildner, Antioxidantien, etc. | q. s. | q. s. | q. s. | q. s. | q. s. |

### Beispiel 1d: Lippengloss

| **Inhaltsstoff (INCI-Bezeichnung)** | **Anteil in Gew.-%** | | |
|---|---|---|---|
| | **Gloss** | **Glossy roll on** | **Gloss Lack** |
| Polyisobuten | ad 100 | ad 100 | |
| Polybuten | | | ad 100 |
| Pentaerythrityl Tetraisostearate | 12,0 | 5,0 | 10,0 |
| Hydrogenated Polydecene | 3,0 | 30,0 | |
| Diisosteraryl Malate | 5,0 | 2,5 | |
| Hydrogenated Polyisobutene | 1,0 | | |
| Myristyl Lactate | 5,0 | | |
| Avocadoöl | 5,0 | | |
| Polyethylenwachs | 1,0 | | 1,0 |
| C30-C50 Fettalkohole | | 0,3 | |
| Trimethylsiloxysilicate | | | 0,3 |
| Triisocetylcitrate | 10,0 | | |
| Octyldodecanol | | | 3,0 |
| Polyglyceryl-2 Tetraisostearate | | | 4,0 |
| Cocoglycerides | | 2,0 | |
| Rizinusöl | | 2,0 | |
| Disteardimonium Hectorite | 0,5 | | 0,75 |
| Propylen Carbonat | 0,12 | | 0,18 |
| Silica Dimethyl Silylate (Aerosil R972) | 0,8 | | 0,6 |
| Calcium Aluminium Borosilikat (z.B. Rona Star Nobel Sparks, Merck) | | | 10,0 |
| Interferenz Pigmente | 10,0 | | |
| Titandioxid CI 77891 | | 2,0 | |
| Eisenoxide CI 77491, 77492, 77499 | | | 0,6 |
| CI 15850 | | 1,3 | |
| CI 15985 | | 0,6 | |
| CI 42090 | | | 0,4 |
| CI 17200 | 0,6 | | |
| Pigment Yellow 216 (Solaplex) | 2,5 | 1,2 | 3,0 |
| Tocopheryl Acetate | 0,5 | 0,5 | 1,0 |
| Parfüm, Konservierungsmittel, Filmbildner, Antioxidantien, etc. | q. s. | q. s. | q. s. |

### Beispiel 1f: Gegossenes Blush

| **Inhaltsstoff (INCI-Bezeichnung)** | **Anteil in Gew.-%** |
|---|---|
| Caprylic / Capric Triglycerides | ad 100 |
| Cera Microcristallina | 3,5 |
| Bienenwachs | 4,0 |
| C20-40 Alkyl Stearat | 6,0 |
| Cetyl Palmitate | 2,5 |
| Isononyl Isononanoate | 6,0 |
| Lauroyl Lysin | 1,0 |
| Nylon | 4,0 |
| Interferenzpigmente | 6,0 |
| Titandioxid CI 77891 | 2,0 |
| Eisenoxide CI 77492, 77499 | 8,0 |
| Pigment Yellow 216 (Solaplex, Johnson Matthey) | 2,0 |
| Cl 15850 | 0,3 |
| PVP/Eicosen Copolymer | 2,0 |
| Tocopheryl Acetate | 1,0 |
| Parfüm, Konservierungsmittel, Antioxidantien, etc. | q. s. |

### 2. Emulsionen

### Beispiel 2a: Emulsionen

| **Inhaltsstoff (INCI-Bezeichnung)** | **Anteil in Gew.-%** | | |
|---|---|---|---|
| | **Emulsions-blush** | **Foundation** | **Getönte Tagescreme** |
| Stearic Acid | 1,5 | 2,0 | |
| Glycerinmonostearat | 2,0 | 1,5 | |
| PEG-100 Stearate | 1,2 | 0,6 | |
| Glyceryl Stearat Citrat | | | 3,5 |
| Octyldodecanol | 7,00 | | 3,0 |
| Cyclomethicone | 6,0 | 12,0 | 3,0 |
| Ceatearyl Alcohol | | | 1,5 |
| Squalane | | | 2,0 |
| Diisostearyl Malate | 2,0 | | |
| Dimethicone/Vinyl Dimethicone Copolymer (z.B. Dow Corning HMW 2220) | 1,00 | | |
| Pentaerythrithyl Tetracocoate | 2,00 | | |
| Shea Butter | | | 5,0 |
| Carbomer | | | 0,6 |
| Xanthan Gum | 0,3 | 0,2 | |
| | | | |
| Magnesium Aluminium Silikate (z.B. Veegum) | 1,0 | 0,8 | |
| Glycerin | 5,0 | 3,0 | 10,0 |
| Pigment Yellow 216 (Solaplex, Johnson Matthey) | 0,3 | 0,6 | 0,3 |
| Glimmer | 1,0 | | |
| Interferenzpigmente | | | 3,0 |
| Eisenoxide | 3,0 | 3,6 | |
| Titandioxid | 2,5 | 5,0 | |
| Talkum | 5,0 | 2,0 | |
| Octyl Methoxy Cinnamat | | | 2,0 |
| mikronisiertes Titandioxid | | | 2,0 |
| Butyl Methoxy Dibenzoylmethan | | | 0,5 |
| Tocopherolacetat | 1,0 | | 1,0 |
| Antioxidantien, Konservierungsmittel, Neutralisationsmittel, Parfum etc. | q. s. | q. s. | q. s. |
| Wasser | ad 100 | ad 100 | ad 100 |

### 3. Puder

### Beispiel 3a: Lidschatten

| **Inhaltsstoff (INCI-Bezeichnung)** | **Anteil in Gew.-%** |
|---|---|
| Talkum | ad 100 |
| Magnesium Stearate | 2,5 |
| Maisstärke | 7,2 |
| Interferenzpigmente (z.B. Timiron Splendid Blue, Merck) | 20,0 |
| Goniochromatische Pigmente (z.B. Xirona Indian Summer, Merck) | 2,0 |
| Pigment Yellow 216 (Solaplex, Johnson Matthey) | 6,0 |
| Glimmer | 3,0 |
| Dimethicone | 2,0 |
| Isopropyl Stearate | 6,0 |
| Cetyl Palmitate | 0,5 |
| Parfüm, Konservierungsmittel, Antioxidantien, etc. | q. s. |

### Beispiel 3b: Puderblush, kompakt

| **Inhaltsstoffe (INCI-Bezeichnung)** | **Anteil in Gew.-%** |
|---|---|
| Talkum | ad 100 |
| Magnesium Stearate | 2,5 |
| Reisstärke | 7,2 |
| Interferenzpigmente | 18,5 |
| Titandioxid Cl 77891 | 2,0 |
| Eisenoxide Cl 77492 | 8,0 |
| Pigment Yellow 216 (Solaplex, Johnson Matthey) | 2,0 |
| Cl 15850 | 0,3 |
| Sphärisches Silica (Ronaspheren, Merck) | 5,0 |
| Dimethicone | 2,0 |
| Isopropyl Stearate | 6,0 |
| Cetyl Palmitate | 0,5 |
| Parfüm, Konservierungsmittel, Antioxidantien, etc. | q. s. |

### 4. Nagellack

### Beispiel 4a: Nagellack

| **Inhaltsstoff (INCI-Bezeichnung)** | **Anteil in Gew.-%** |
|---|---|
| Ethyl Acetate | ad 100 |
| Butyl Acetate | 30,0 |
| Nitrocellulose | 10,0 |
| Isopropyl Alcohol | 6,0 |
| Tosylamide / Formaldehyde Resin | 7,5 |
| Dibutyl Phthalate | 3,0 |
| Camphor | 2,0 |
| Acrylate Copolymer | 5,0 |
| BiOCl | 2,0 |
| Goniochromatische Pigmente (z.B. Xirona Indian Summer, Merck) | 0,3 |
| Pigment Yellow 216 (Solaplex, Johnson Matthey) | 2,0 |
| Stearalkolium Hectorite | 1,2 |
| Antioxidantien, etc. | q. s. |

## Patentansprüche

1. Kosmetische Zubereitung zur dekorativen Anwendung auf Haut oder Nägeln, welche mindestens eine feste Phase enthält, die anorganische Pigmente aufweist, welche Titan-, Zinn- und Zinkmischoxide enthalten, wobei die molaren Mengenverhältnisse der Oxide der Summenformel (TiO₂)ₐSnO(ZnO)_{b}(SnO₂)_{c} entsprechen, worin a eine Zahl zwischen 0,3 und 6,2, b eine Zahl zwischen 0,04 und 6,2 und c eine Zahl zwischen 0 und 7 ist.

2. Kosmetische Zubereitung nach Anspruch 1, **dadurch gekennzeichnet, dass** die Pigmente (Gelbpigmente) Strahlung mit einer Wellenlänge von 400 bis 450 nm absorbieren, so dass die tatsächlich beobachtete, reflektierte Farbe (Körperfarbe) im Gelbbereich liegt.

3. Kosmetische Zubereitung nach Anspruch 2, **dadurch gekennzeichnet, dass** die Zubereitung 0,01 bis 20 Gew.-% der Gelbpigmente enthält.

4. Kosmetische Zubereitung nach Anspruch 2, **dadurch gekennzeichnet, dass** die Zubereitung 0,05 bis 15 Gew.-% der Gelbpigmente enthält.

5. Kosmetische Zubereitung nach Anspruch 3, **dadurch gekennzeichnet, dass** die Zubereitung 0,1 bis 12 Gew.-% der Gelbpigmente enthält.

6. Kosmetische Zubereitung nach einem der Ansprüche 1 bis 5, **dadurch gekennzeichnet, dass** die Zubereitung neben der festen Phase eine weitere hydrophobe oder hydrophile Phase enthält.

7. Kosmetische Zubereitung nach Anspruch 6, **dadurch gekennzeichnet, dass** die Zubereitung zusätzlich noch weitere Phasen enthält, welche hydrophob und/oder hydrophil sind.

8. Kosmetische Zubereitung nach Anspruch 6 und 7, **dadurch gekennzeichnet, dass** die hydrophobe Phase organische Lösungsmittel enthält.

9. Kosmetische Zubereitung nach Anspruch 8, **dadurch gekennzeichnet, dass** die Zubereitung zusätzlich Filmbildner, Weichmacher, Glanz- und Haftverbesserer enthält.

10. Kosmetische Zubereitung nach Anspruch 6 und 7, **dadurch gekennzeichnet, dass** die hydrophile Phase Wasser, einwertige Alkohole, Polyole oder deren Mischungen enthält.

11. Kosmetische Zubereitung nach Anspruch 10, **dadurch gekennzeichnet, dass** die hydrophile Phase zusätzlich Gelbildner enthält.

12. Kosmetische Zubereitung nach Anspruch 6 und 7, **dadurch gekennzeichnet, dass** die hydrophobe Phase ein kosmetisch verwendbares Öl ist.

13. Kosmetische Zubereitung nach Anspruch 12, **dadurch gekennzeichnet, dass** das Öl aus der Gruppe enthaltend
aliphatische Kohlenwasserstoffe mit einer Kettenlänge von 6 bis 22 Kohlenstoffatomen,
Ester gesättigter und ungesättigter Carbonsäuren, die eine Kettenlänge von 3 bis 30 Kohlenstoffatomen aufweisen, und gesättigten und ungesättigten Alkoholen, die eine Kettenlänge von 2 bis 30 Kohlenstoffatomen und 1 bis 5 Hydroxygruppen aufweisen, Ether aus gesättigten oder ungesättigten Alkoholen, die eine Kettenlänge von 2 bis 30 Kohlenstoffatomen und 1 bis 5 Hydroxygruppen aufweisen,
Ester der Kohlensäure mit Alkoholen, die eine Kettenlänge von 2 bis 30 Kohlenstoffatomen und 1 bis 5 Hydroxygruppen aufweisen,
cyclischen und linearen Silikonölen sowie
Estern aromatischer Carbonsäuren und gesättigten und ungesättigten Alkoholen, die eine Kettenlänge von 2 bis 30 Kohlenstoffatomen und 1 bis 5 Hydroxygruppen aufweisen, gewählt ist.

14. Kosmetische Zubereitung nach Anspruch 7, **dadurch gekennzeichnet, dass** die Zubereitung zusätzlich mindestens einen Emulgator enthält.

15. Kosmetische Zubereitung nach einem der Ansprüche 1 bis 14, **dadurch gekennzeichnet, dass** die Zubereitung weitere feste Bestandteile enthält.

16. Kosmetische Zubereitung nach Anspruch 15, **dadurch gekennzeichnet, dass** die festen Bestandteile Wachse sind.

17. Kosmetische Zubereitung nach Anspruch 16, **dadurch gekennzeichnet, dass** die Wachse aus der Gruppe bestehend aus Bienenwachsen, Teilfraktionen des Bienenwachs und Bienenwachsderivaten, mikrokristallinen Wachsen, Ceresin, Ozokeriten, Carnaubawachs, Candelillawachs, Reiswachs, Japanwachs, Schellackwachs sowie homopolymeren Polyethylenen ausgewählt sind.

18. Kosmetische Zubereitung nach Anspruch 16 und 17, **dadurch gekennzeichnet, dass** die Zubereitung zusätzlich Öle enthält.

19. Kosmetische Zubereitung nach Anspruch 15, **dadurch gekennzeichnet, dass** die festen Bestandteile pulverförmige Mineralien sind.

20. Kosmetische Zubereitung nach Anspruch 19, **dadurch gekennzeichnet, dass** das Mineral Talkum ist.

21. Kosmetische Zubereitung nach Anspruch 20, **dadurch gekennzeichnet, dass** die Zubereitung zusätzlich weitere pulverförmige Mineralien, Weißpigmente, Haftverbesserungsmittel und Gleitmittel enthält.
